# EUROPEAN PATENT APPLICATION

(11) **EP 3 842 039 A1**
(43) Date of publication of application: **30.06.2021**
(21) Application number: 19219673.1
(22) Date of filing: 24.12.2019
(51) Int. Cl.: A61K 31/05, A61K 31/192, A61K 9/48, A61K 31/352, A61K 31/353, A61K 31/575, A61K 36/03, A61K 36/185, A61P 25/28, A61K 45/06, A23L 33/00

(54) **FOOD SUPPLEMENT COMPRISING CANNBINOIDS AND PHYTOSTEROLS FOR ALZHEIMER**

(71) Applicant: Folium Biosciences Europe B.V., 1382 GS Weesp (NL)
(72) Inventor: Woerlee, Geert Feye, 1382 GS WEESP (NL); Trambitas, Daniela Oana, 1382 GS Weesp (NL)
(74) Representative: van Essen, Peter Augustinus

(57) **Abstract**

The present invention is in the field of a Food supplement for Alzheimer. Alzheimer's disease (AD), and likewise associated or similar discomforts, is a chronic neurodegenerative disease that usually starts slowly and gradually worsens over time. The present invention provides a food supplement for preventing Alzheimer's disease, for delaying Alzheimer's disease, for decline of ageing, or combinations thereof.

## Description

### FIELD OF THE INVENTION

The present invention is in the field of a Food supplement for Alzheimer. Alzheimer's disease (AD), and likewise associated or similar discomforts, is a chronic neurodegenerative disease that usually starts slowly and gradually worsens over time. The present invention provides a food supplement for preventing Alzheimer's disease, for delaying Alzheimer's disease, for decline of ageing, or combinations thereof.

### BACKGROUND OF THE INVENTION

Deficits in learning and memory problems are generally associated with ageing. A most consistent change during healthy ageing in human's brain structure is shrinkage in brain volume and expansion of the ventricular system. There is substantial evidence suggesting that the activity of the brain endocannabinoid system (ECS) declines with ageing, which is considered due to CB1 receptor expression and coupling to G proteins being reduced in the brain tissue of mammals which are getting older.

In some case, this decline is even promoted by additional neurodegenerative disorders, such as Alzheimer (AD). The AD may be signalled by cognitive impairment in early life stages. This disease is characterized by a progressive neurological disorder which is considered to be provoked mainly by the deposition of Amyloid-β deposits in the senile plaques, neurofibrillary tangles, neuroinflammation which leads to loss of brain volume and a progressive decline in brain functions, particularly memory. A key role in the development of the disease is attributed to the disturbance of the cholesterol-transport in the brain, which lead progressively to the AD.

Thus, a better understanding of such processes may lead to prevention of the molecular and cellular degradation processes, offering novel routes for therapy and/or prevention.

Recent publications have reported clinical researches which evidenced that exposure to chronic low dosage of Δ⁹-tetrahydrocannabidiol (THC) may reverse practically the age-related decline in cognitive performances. THC has been found also to promote the removal of the toxic clumps of Amyloid-β deposits in the brain. The memory seems to be improved when brain cholesterol-transport is activated or re-activated and AD effects are evidently diminished. However, the THS is found to be partly active only.

It is considered that alternatives to this exposure or improvements thereof may be needed.

The present invention therefore relates to a food supplement, a dosage comprising said food supplement, and a medicament, which solve one or more of the above problems and drawbacks of the prior art, providing reliable results, without jeopardizing functionality and advantages.

### SUMMARY OF THE INVENTION

The present invention relates to a food supplement for Alzheimer wherein the supplement comprises 0.1-90 wt.% of at least one active cannabinoid, preferably 0.5-60 wt.%, more preferably 1-35 wt.%, even more preferably 2-20 wt.%, such as 5-10 wt.%, wherein the cannabinoid is of phyto, endo, or synthetic origin, 0.1-90 wt.% of at least one active phytosterol, preferably 0.5-60 wt.%, more preferably 1-35 wt.%, even more preferably 2-20 wt.%, such as 5-10 wt.%, wherein the phytosterol is extracted from biological species, such as from algae, or a pharmaceutically acceptable salt, or a pharmaceutically acceptable derivative thereof, or a tautomer thereof, or a solvate thereof.

It has been found that one way to activate the brain cholesterol-transport is to make use of phytosterols, which can take over the process, and can be up-taken easily by the human body. In this way one avoids the negative impact of some synthetic activators, which can induce sever health side effects. Phytosterols considered are the Saringosterols and fucosterols, which may be found in specific macro algae, typically of the order Fucales, and preferably of the family Sargassaceae, such as Sargassum Seaweed, and of the order Laminariales, specifically of the family Lassoniaceae, such as Ecklonia species, such as *Ecklonia cava, and Ecklonia stolonifera.* The ratio Saringosterols/Fucosterols may be between 0.01 to 0.8, depending on a type of the seaweed used for extraction. These seaweeds are however not very tasty and have a strong odour. The uptake of this Saringosterols and fucosterols, and especially 24(S)-Saringosterol, and (3S,8S,9S,10R,13R,14S,17R)-10,13-Dimethyl-17-[(E,2R)-5-propan-2-ylhept-5-en-2-yl]-2,3,4,7,8,9,11,12,14,15,16,17-dodecahydro-1H-cyclopenta[a]phenanthren-3-ol, is found to diminish the Amyloid-β depositions up to 70%, and is found to help restore neurological behaviours without registering any side effects caused normally, such as by the synthetic LXRα/β activators. Inventors have found a clear benefit of having a diet rich in phytosterols, such as obtained from Seaweed, combined with a controlled low dosage of Δ⁹-tetrahydrocannabidiol (THC). It is an advantage that relatively low dosages can be used. The above combination may be considered, however in practice it is found to be rather difficult to accomplish, as a minimum amount of seaweed which should be part of a daily dietary ration should be around ten grams. Apart from the fact that not all seaweed types contain this preferred saringosterol, such as Sargassumsterol, the availability of these seaweeds may also be rather rare, whereas a concentration of the phytosterol in the seaweed may also be limited.

Distribution of the cannabinoids is also very limited and well controlled in a lot of countries. It is noted that their activity can be easily reduced, such as by utilizing a wrong extraction procedure, or improper purification. A food supplement containing both active ingredients (in high purity) in specific ratios could be a welcome approach to be utilized by elderly persons or for those diagnosed with AD disease, such as in early stages of the disease.

Both claimed active components have biological origin and inventors have found that these can be easily extracted by using supercritical CO₂. It has been found that high purity compounds are obtained, with virtually no impurities, such as < 1 wt.%, in active form, and of good quality. The pressurized gas process is found to deliver natural extracts free of chemical solvents, and can therefore be used directly as food supplements, such as in combination with edible oils or fats, or by encapsulation utilizing edible bio-degradable capsules originated in natural polymers like polysaccharides or waxes, fats, or mixtures thereof. Also, it is found that natural actives extracted from algae together have a synergic positive effect when utilized as such without any post fractionation. The loading of the actives in the capsule material can be between 0.1 and 60% and the ratio between the cannabidiols and the phytosterols can also vary between 0.1 to 100%.

The active cannabinoid is of phyto origin, endo origin, or synthetic origin. It is extracted from biological species, typically the Cannabaceae family, preferably the genus Cannabis, such as *Cannabis sativa, Cannabis indica, Cannabis ruderalis.* The phytosterol is extracted from biological species, such as for Fucales, preferably from brown algae such as the Sargassaceae family, such as from the *Sargassum* genus.

In a second aspect the present invention relates to a method of providing a food supplement according to the invention, comprising a critical CO₂ extraction method. In the method pre-dried milled seaweed is used, which is contacted with CO₂ near supercritical conditions. The extraction takes place under controlled temperature and pressure. The seaweed extract may contain a mixture of Saringosterols and Fucosterols in approximate ratios of 0.01 to 0.8 Saringosterol/Fucosterol. When using this extract, the daily intake, depending on the Saringosterol concentration in the pure extract, can be reduced to only 10-35 mg extract/day. This extract complies with a daily recommended concentration of 600-700 µg Saringosterol, so about 2-6 wt.% saringosterol. On top of this the extract is also rich in Fucosterol.

In a third aspect the present invention relates to a dosage comprising a food supplement according to the invention, comprising 0.1-60 wt.% food supplement, and 1-1000 mg of as active ingredient, wherein the active ingredient comprises at least one active cannabinoid, wherein the cannabinoid is of phyto, endo, or synthetic origin, and at least one active phytosterol, wherein the phytosterol is extracted from biological species, preferably 2-500 mg active ingredient, more preferably 5-200 mg active ingredient, such as 10-100 mg active ingredient. A recommended dose now is approximately 2-3.5g/day of dry seaweed (Sargassum) or 600-700 micrograms/day pure extract in combination with cannabinoids

In a fourth aspect the present invention relates to a food supplement according to the invention, for use as a medicament for preventing Alzheimer's disease, for delaying Alzheimer's disease, for decline of ageing, for restoring brain tissue, for treatment of a person with a genetically increased chance for Alzheimer's disease, or combinations thereof.

Thereby the present invention provides a solution to one or more of the above-mentioned problems and drawbacks.

Advantages of the present description are detailed throughout the description.

Use of the verb "to comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage.

The invention further pertains to a method or process comprising one or more of the characterising features described in the description and/or shown in the attached drawings. The various aspects discussed in this patent can be combined in order to provide additional advantages. Furthermore, some of the features can form the basis for one or more divisional applications.

### DETAILED DESCRIPTION OF THE INVENTION

The present invention relates in a aspect to a food supplement according to claim 1.

In an exemplary embodiment the present food supplement may be for preventing Alzheimer's disease, for delaying Alzheimer's disease, for decline of ageing, or combinations thereof.

In an exemplary embodiment of the present food supplement the cannabinoid may be selected from Tetrahydrocannabinolic acid THCA, Cannabidiolic acid CBDA, Cannabigerolic Acid CBGA, cannabichromenic acid CBCA, cannabinol acid (CBNA), tetrahydrocannabivarin acid (THCVA), cannabielsoin acid (CBEA), cannabicycol acid (CBLA), cannabicitran acid (CBTA), there iso-forms, their cyclic forms, derivatives thereof, and their active neutral forms, such as Δ⁹-tetrahydrocannabidiol (THC).

In an exemplary embodiment of the present food supplement the phytosterol may be selected from saringosterols, preferably Sargassum Seaweed saringosterol, especially 24(S)-saringosterol.

In an exemplary embodiment of the present food supplement the at least one cannabinoid and phytosterols are each individually >90% pure, preferably > 99% pure.

In an exemplary embodiment of the present food supplement a total amount of active cannabinoid and active phytosterol may be between 0.1-60 wt.%, preferably between 0.5-40 wt.%, more preferably between 1-30 wt.%, such as between 2-15 wt.%.

In an exemplary embodiment of the present food supplement the at least one cannabinoid and phytosterols may each individually be extracted by super-critical CO₂.

In an exemplary embodiment of the present food supplement the food supplement may comprise <5 ppm solvent, preferably <2ppm solvent, such as <1ppm solvent.

In an exemplary embodiment the present food supplement may comprise 5-50wt.% of edible oil, and edible fat, preferably 10-25 wt.%.

In an exemplary embodiment the present food supplement may be provided in a capsule, where the capsule material is selected from polysaccharides, waxes, fats, proteins, and mixture thereof. The capsule is found to improve a release profile and may provide a taste masking effect in addition.

In an exemplary embodiment of the present food supplement a ratio between weights of cannabinoid and phytosterol may be from 1:1000 to 1000:1, preferably from 1:200 to 200:1, more preferably from 1:100 to 100:1, even more preferably from 1:30 to 30:1, such as from 1:10 to 10:1.

In an exemplary embodiment the present food supplement may further comprise at least one carrier selected from oils, powders, and mixtures thereof. Plant material like Seaweed can be also considered to be encapsulated as it is.

In an exemplary embodiment of the present food supplement may further comprise one or more of a pharmaceutically acceptable excipient, such as selected from fillers, binders, and disintegrants. A disintegrant is any material that may be added to the present food supplement to make it disintegrate, and thereby releasing the active ingredient, typically on contact with water.

The one or more of the above examples and embodiments may be combined, falling within the scope of the invention.

### EXAMPLES

The below relates to examples, which are not limiting in nature, showing the benefits of the invention.

| **Running time [min]** | **Sample [mg]** | **Solvent mixtured [ml]** | **Fuco [µg]** | **24R,S-Saringo [µg]** | **Fuco [µg/mg]** | **24R,S-Sariongo µg/mg** | **Saringo/Fuco** |
|---|---|---|---|---|---|---|---|
| 60 | 900 | 10 | 73 | 17.021 | 81.442 | 18,912 | 0.2322 |
| 120 | 1100 | 10 | 78 | 16.620 | 70.650 | 15.109 | 0,213857471 |
| 180 | 800 | 10 | 60 | 13.850 | 75.310 | 17.313 | 0,229881741 |
| 240 | 700 | 10 | 38 | 8.890 | 53.749 | 12.700 | 0,236281671 |

10 ml of CCl₃/MeOH was used each time. 10 µl of worked-up material was obtained.

The invention is further detailed by the accompanying figures, which are exemplary and explanatory of nature and are not limiting the scope of the invention. To the person skilled in the art it may be clear that many variants, being obvious or not, may be conceivable falling within the scope of protection, defined by the present claims.

### FIGURES

The invention although described in detailed explanatory context may be best understood in conjunction with the accompanying figures.
Fig. 1a-b show a Saringosterol and a fucosterol.
Fig. 2 shows Focus veziculosus.
Fig. 3 shows an experimental set-up.
Fig. 4 shows milled seaweed.
Fig5a,b Seaweed extract collected from separator.
Figs. 6a-c show Extract and CBD, Coating phase, and Emulsion.
Fig. 7 shows dry powder.
Fig. 8a,b show SEM micrographs (different magnifications of the coated particles.
Fig. 9: drying process.
Figs. 10a,b obtained products.

### DETAILED DESCRIPTION OF THE FIGURES

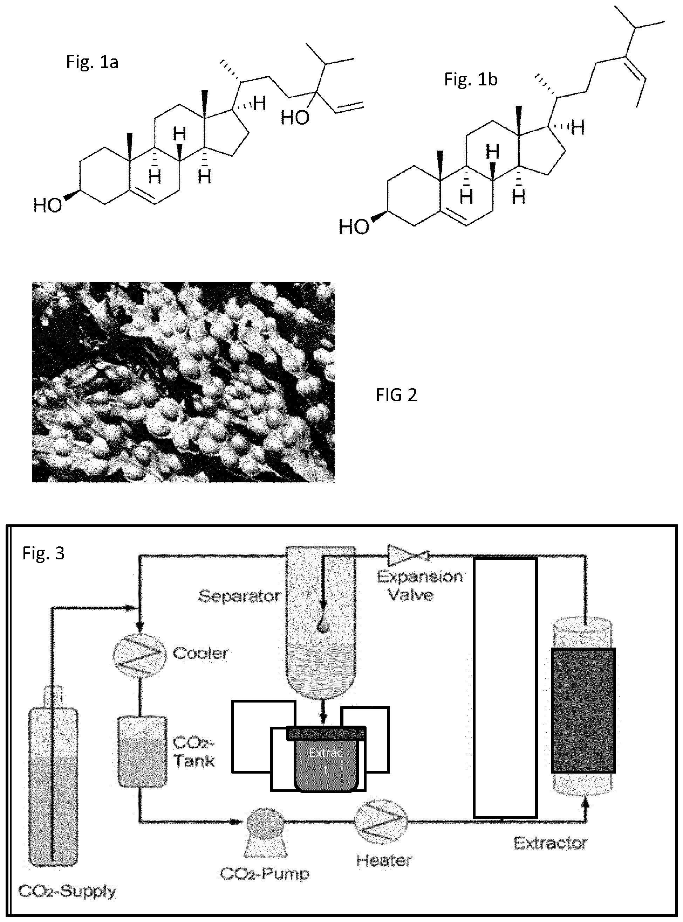

Fig. 1a-b show a Saringosterol (PubChem CID 14161394) and a fucosterol (CAS 17605-67-3).

### Seaweed extraction tests

### Example 1.

Tested Material: Bladder wrack known also as rockweed or bladder fucus (Focus veziculosus) harvested form North Sea (see fig. 2).

It is currently used as a dietary supplement as an accelerator for basal metabolism or as a mean to muscle recovery from intensive physical training. It also helps weight loss and its control.

Due to its rich extracts like fucoidans, alginic acids, and laminarin has wide recommendations for its positive results in reducing blood glucose levels, or reducing the plasma cholesterol levels. It has a high content of iodine which give a certain restriction for usage specially for people with thyroid disorders, hypertension, bleeding problems or pregnant women. However, the iodine content recommends this seaweed also for hypo-thyroidism.

### Milled seaweed :400g

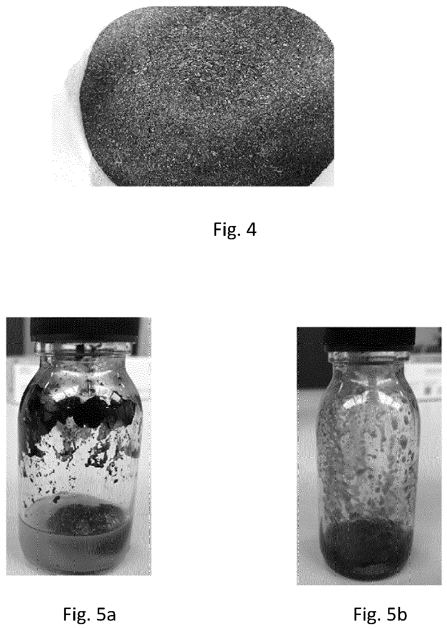

Super critical (Sc) CO₂ extraction setup was used for extracting the active ingredients from this seaweed (see fig. 3). 400g of milled dry algae is placed into a 1L extractor and CO2 under supercritical conditions is brought in contact with the algae. The active ingredients from the algae are solubilized into the scCO₂ at operating conditions (30000 kPa (300 bar), 40 °C, for 4h). And the CO₂ flow rich in extract is passed into the separator where because of solubility differences (due to pressure differences) the extracts are separated from the scCO₂ and further collected in the extract vessel (fig. 5a,b).

The extract contains Fucosterol and Saringosterol (rations S/F = 0.23). The extract contains also some water which is co-extracted together with the sterol fraction.

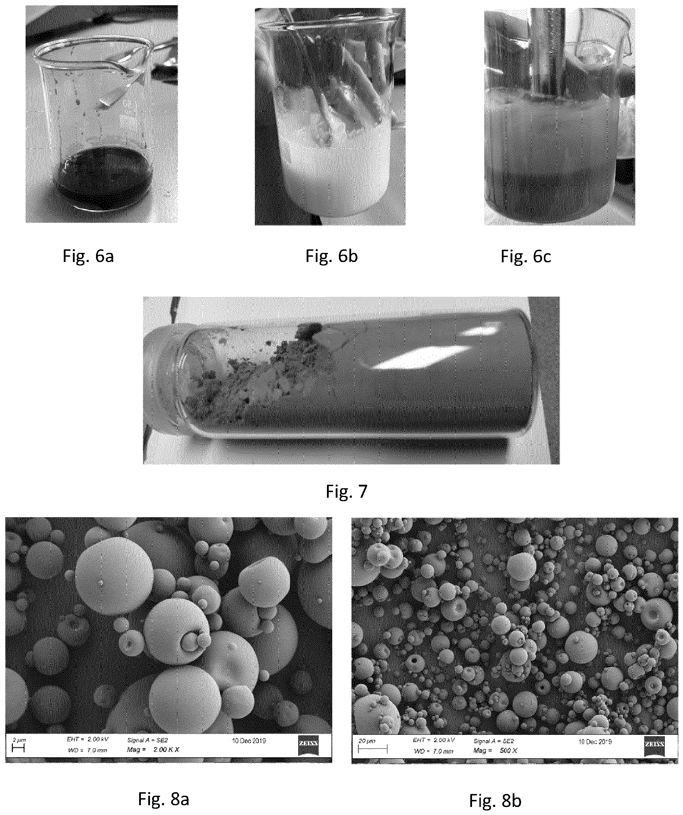

The water fraction is removed and the sterol fraction is mixed with CBD supplied via separate extraction form hemp flowers (following the present procedure) a coating material containing modified starch and maltodextrin (Capsule:MD5:MD20). The ration used in this example was: approx. 87% coating comprise of approximately 35% MD20, 8.7%MD5, and 43.5%Capsule. The loading used was approximately 13% , comprising of 10.4% seaweed extract (with a ration Saringosterol/Fucosterol of 0.23) and 2.5% CBD (99%purity) extracted also by scCO₂ from dry hemp flowers.
The two phases are mixed to form emulsion. Figs. 6a-c show Extract and CBD, Coating phase, and Emulsion. The mixture coating /extract forms a stable emulsion which is further used to dry it by scCO₂ spraying process.

Dry powder (fig. 7) consists of encapsulated extracts and CBD in a coated matrix of Modified starch (Capsule) and maltodextrin (MD20 and MD5).

During the drying process, based on the material crystallization properties, the coating matrix will form around the extract mixture a solid thin protective layer which entrap the sterols and CBD inside the capsule material.

SEM micrographs (figs. 8a,b) show different magnifications of the coated particles.

The encapsulation efficiency can also be computed based on PL/PT determinations (Journal of AOAC International, 2005, Vol 88, pg 1271-1274). PL/PT stands for active ingredient found on the exterior of the particles and total active ingredients fund in a specific amount of encapsulated powder.

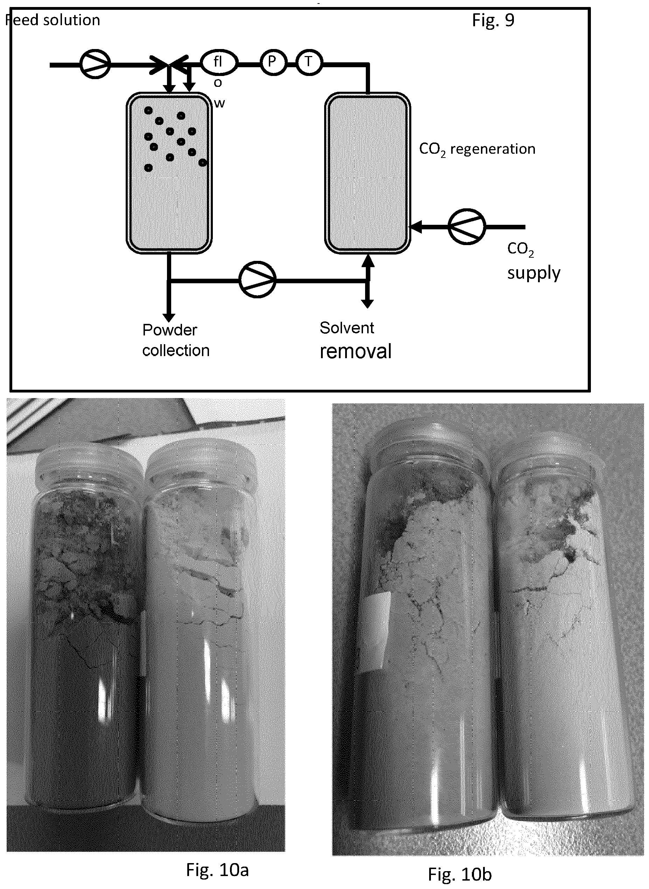

The spray drying process is done in a closed system with CO₂ continuous recirculation (fig. 9), and Schematic presentation of the encapsulation process.

### Example 2.

Same extraction process as in example 1.

For loading we used only seaweed extract without CBD.

The product is lighter in colour compared to the one produced with CBD. Figs. 10a,b show obtained products.

## Claims

1. Food supplement for Alzheimer wherein the supplement comprising
0.1-90 wt.% of at least one active cannabinoid, preferably 0.5-60 wt.%, more preferably 1-35 wt.%, even more preferably 2-20 wt.%, such as 5-10 wt.%, wherein the cannabinoid is of phyto, endo, or synthetic origin, wherein the cannabinoid is extracted from biological plant species,
0.1-90 wt.% of at least one active phytosterol, preferably 0.5-60 wt.%, more preferably 1-35 wt.%, even more preferably 2-20 wt.%, such as 5-10 wt.%, wherein the phytosterol is extracted from biological species, such as from algae,
or a pharmaceutically acceptable salt, or a pharmaceutically acceptable derivative thereof, or a tautomer thereof, or a solvate thereof,
wherein all wt.% are based on a total weight of the food supplement.

2. Food supplement according to embodiment 1, wherein the food supplement is for preventing Alzheimer's disease, for delaying Alzheimer's disease, for decline of ageing, or combinations thereof.

3. Food supplement according to any of embodiments 1-2, wherein the cannabinoid is selected from Tetrahydrocannabinolic acid (THCA), Cannabidiolic acid (CBDA), Cannabigerolic Acid (CBGA), cannabichromenic acid (CBCA), cannabinol acid (CBNA), tetrahydrocannabivarin acid (THCVA), cannabielsoin acid (CBEA), cannabicycol acid (CBLA), cannabicitran acid (CBTA), there iso-forms, their cyclic forms, derivatives thereof, and their active neutral forms, such as Δ⁹-tetrahydrocannabidiol (THC).

4. Food supplement according to any of embodiments 1-3, wherein the phytosterol is selected from saringosterols, preferably Sargassum Seaweed saringosterol, especially 24(S)-saringosterol.

5. Food supplement according to any of embodiments 1-4, wherein the at least one cannabinoid and phytosterols are each individually >90% pure, preferably > 99% pure, and/or wherein a total amount of active cannabinoid and active phytosterol is between 0.1-60 wt.%, preferably between 0.5-40 wt.%, more preferably between 1-30 wt.%, such as between 2-15 wt.%.

6. Food supplement according to any of embodiments 1-5, wherein the at least one cannabinoid and phytosterols are each individually extracted by super-critical CO₂.

7. Food supplement according to any of embodiments 1-6, wherein the food supplement comprises <5 ppm solvent.

8. Food supplement according to any of embodiments 1-7, comprising 5-50wt.% of edible oil, and edible fat.

9. Food supplement according to any of embodiments 1-8, in a capsule, where the capsule material is selected from polysaccharides, waxes, fats, proteins, and mixture thereof.

10. Food supplement according to any of embodiments 1-9, wherein a ratio between weights of cannabinoid and phytosterol is from 1:1000 to 1000:1, preferably from 1:200 to 200:1, more preferably from 1:100 to 100:1, even more preferably from 1:30 to 30:1, such as from 1:10 to 10:1.

11. Food supplement according to any of embodiments 1-10, further comprising at least one carrier selected from oils, powders, and mixtures thereof.

12. Food supplement according to any of embodiments 1-11, further comprising one or more of a pharmaceutically acceptable excipient, such as selected from fillers, binders, and disintegrants.

13. Method of providing a food supplement according to any of embodiments 1-12, comprising super critical CO₂ extraction under increased pressure of > 10 kPa, a temperature of >20 °C, during a period of > 1h.

14. Dosage comprising a food supplement according to any of embodiments 1-12, comprising 0.1-60 wt.% food supplement, and 1-1000 mg of as active ingredient, wherein the active ingredient comprises at least one active cannabinoid, wherein the cannabinoid is of phyto, endo, or synthetic origin, and at least one active phytosterol, wherein the phytosterol is extracted from biological species, preferably 2-500 mg active ingredient, more preferably 5-200 mg active ingredient, such as 10-100 mg active ingredient.

15. Food supplement according to any of embodiments 1-12, for use as a medicament for preventing Alzheimer's disease, for delaying Alzheimer's disease, for decline of ageing, for restoring brain tissue, for treatment of a person with a genetically increased chance for Alzheimer's disease, or combinations thereof.
